# FASCICULE DE BREVET EUROPEEN

(11) **EP 0 369 324 B1**
(45) Date de publication et mention de la délivrance du brevet: **02.11.1995**
(21) Numéro de dépôt: 89120771.4
(22) Date de dépôt: 09.11.1989
(51) Int. Cl.: A61B 17/10

(54) **Instrument de chirurgie**
Chirurgisches Instrument
Surgical instrument

(30) Priorité: 11.11.1988 CH 4176/88
(43) Date de publication de la demande: 23.05.1990
(73) Titulaire: United States Surgical Corporation, Norwalk, Connecticut 06856 (US)
(72) Inventeur: De Salis, Sker Jean-Rodolphe, CH-20006 Neuchâtel (CH); Klaiber, Christian, CH-3270 Aarberg (CH)
(74) Mandataire: Marsh, Roy David

(56) Documents cités:
- EP-A- 0 140 552
- FR-A- 2 310 117
- GB-A- 1 452 185
- GB-A- 2 048 685
- JP-A-51 149 985
- US-A- 3 593 903
- US-A- 4 784 137

## Description

L'invention a pour objet un instrument de chirurgie selon le préambule de la revendication 1, pour chasser des éléments de fixation dans le tissu d'un corps.

Un instrument de chirurgie selon le préambule de la revendication 1 est décrit dans le brevet japonais JP-A-51-149985. Ce document se réfère à un instrument de piqûre, à être employé dans un passage d'accessoires d'un endoscope flexible. L'instrument de piqûre comprend une plaque de support mobile ainsi qu'une structure à tenir des agrafes à former en s'y appuyant. Dès que la plaque de support est fermée sur de tissu, p.ex. un polype, un instrument coupeur est déplacé du côté distal moyennant un poussoir afin de piquer et détacher le polype.

La présente invention a également pour objet un instrument de chirurgie dessiné à l'ablation d'organes ou de parties d'organes, notamment par des opérations de pincement, d'agrafage et de coupe des tissus par accès endoscopique.

Le procédé classique d'intervention chirurgicale mis en oeuvre pour l'ablation d'organes consiste à faire une incision, sur une longueur relativement grande, à opérer sur l'organe défaillant en fonction du besoin, puis à suturer.

On utilise de plus en plus pour la suture des appareils permettant l'agrafage au lieu de coudre à la main.

Un dispositif permettant par exemple ce genre d'agrafage est décrit dans le brevet US-A-4 429 695.

Ce dispositif comporte deux mâchoires, respectivement fixe et mobile, la mâchoire mobile étant articulée au niveau de son extrémité arrière sur l'extrémité libre correspondante d'une structure réceptrice, dans laquelle est logée la mâchoire fixe.

Par ailleurs, ces deux mâchoires peuvent être bloquées, l'une par rapport à l'autre, à l'aide d'une poignée qui est articulée sur la partie médiane de la mâchoire mobile et qui est susceptible de venir s'engager, par l'intermédiaire d'ouvertures débouchantes formant came, sur des tenons latéraux solidaires de la structure réceptrice.

Toutefois, ce dispositif ne donne pas entière satisfaction puisqu'il est de dimension telle qu'il faut pratiquer des incisions importantes qui laissent des cicatrices gênantes et peu esthétiques.

Ainsi, on cherche désormais à utiliser pour ce type d'intervention la technique dite endoscopique (laparoscopie, thoracoscopie, etc...).

Cette technique d'intervention consiste à pratiquer, à l'aide d'un trocart, par exemple dans la paroi de l'abdomen, plusieurs orifices les plus petits possibles, à insérer dans certains de ces orifices des instruments de préhension ou d'observation, tel qu'un endoscope, puis à insérer dans d'autres orifices un ou plusieurs intruments de chirurgie, tous ces instruments coulissant au travers de tubes de passages de l'extérieur de la paroi jusqu'à l'organe défaillant.

Cette technique permet de réduire de façon considérable la grandeur de l'incision. Cependant, faute d'un instrument adapté et suffisamment miniaturisé, son champ d'application est actuellement limité, elle exige une grande habileté du chirurgien et nécessite un temps d'intervention assez long.

Il est ainsi le but de l'invention de proposer un instrument d'agrafage endoscopique qui permet au chirurgien le degré de commande recherché, ainsi qu'une possibilité de fermer et relâcher, en répétition, les mâchoires, afin de faire l'épreuve, jusqu'à ce que le chirurgien est satisfait de la réalisation correcte et précise de l'opération de fermeture des mâchoires.

Ce but est atteint moyennant un instrument de chirurgie, qui présente les caractéristiques définies dans la revendication 1.

Selon une caractéristique particulière de l'invention, la mâchoire mobile a la forme d'un levier coudé dont un bras forme la mâchoire proprement dite, tandis que l'autre bras est articulé de préférence à la jonction entre la mâchoire fixe et l'extrémité avant du corps de l'instrument.

Selon une autre caractéristique, le moyen de commande de la mâchoire mobile est constitué par une tige rigide attelée à cette mâchoire, au proche de la jonction entre les deux bras précités, cette tige étant logée à l'intérieur du corps de l'instrument dans lequel elle peut coulisser.

L'invention sera mieux comprise à la lecture de la description détaillée qui suit, prise en référence aux dessins annexés qui sont donnés uniquement à titre d'exemple, et dans lesquels :
- la figure 1 est une vue de côté et partiellement en coupe de l'instrument selon l'invention;
- la figure 2 est une vue en coupe transversale faite suivant la ligne II-II de la figure 1 et dans laquelle est représenté en traits interrompus un couteau pouvant coulisser le long du corps de l'instrument;
- la figure 3 est une vue en perspective éclatée de l'instrument selon l'invention, dans laquelle les dimensions relatives des différents éléments n'ont pas été strictement respectées; et
- les figures 4 à 9 représentent respectivement différentes phases d'une intervention chirurgicale menée avec l'instrument des figures 1 à 3.

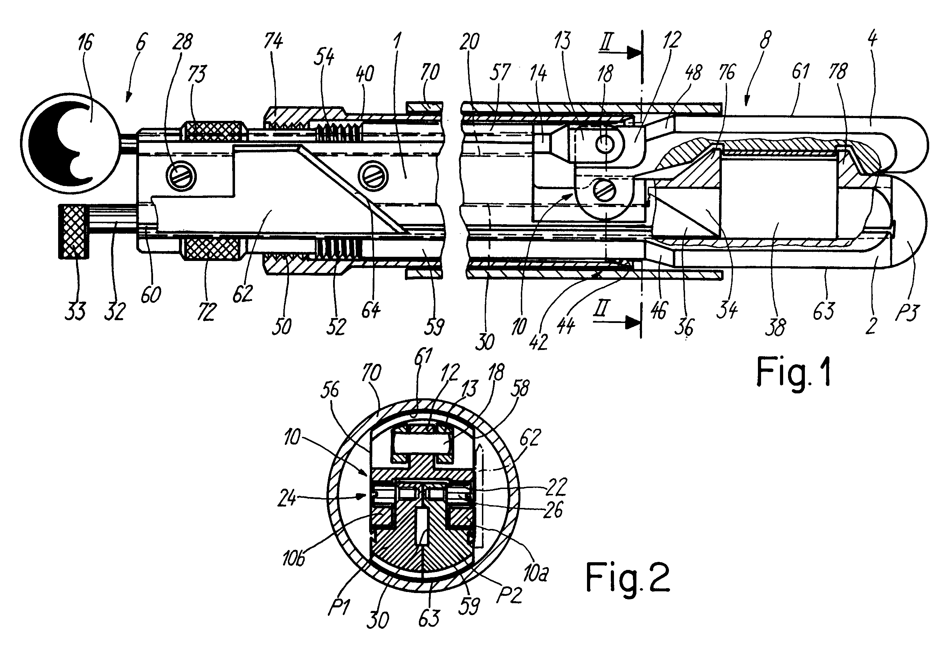

Comme il est représenté sur les figures 1 à 3, l'instrument de chirurgie selon l'invention comporte un corps ou partie réceptrice 1 pourvu à son extrémité avant de deux mâchoires respectivement fixe 2 et mobile 4, articulées l'une par rapport à l'autre.

La mâchoire mobile 4, qui peut être actionnée par des moyens de commande 6 débouchant à l'extrémité arrière du corps 1, présente la forme d'un levier coudé dont un bras 8 comporte les parties actives de pincement des tissus et forme la mâchoire proprement dite.

L'autre bras 10 de ce levier coudé est articulé à la jonction entre la mâchoire fixe 2 et l'extrémité avant du corps 1, sur la partie arrière de la mâchoire fixe 2.

Comme on le voit de la figure 2, le bras d'articulation 10 de la mâchoire mobile 4, qui forme la partie arrière de celle-ci , présente en direction transversale la forme d'un "U" renversé, de la base duquel s'élève une nervure de liaison 12.

Cette nervure de liaison 12 reçoit, par un montage en chape, un étrier 13 ménagé dans le prolongement d'une tige rigide 14 qui constitue, avec un embout sphérique 16 vissé sur celle-ci, les moyens de commande 6 de la mâchoire mobile 4.

De ce fait, la tige 14 est attelée à la mâchoire mobile 4, au proche de la jonction entre les deux bras 8 et 10, par l'intermédiaire d'un axe 18 reliant de façon articulée l'étrier 13 à la nervure 12.

Par ailleurs, la tige 14 est logée à l'interieur du corps 1, dans un orifice débouchant 20 de sorte que cette tige 14 peut coulisser librement dans le corps 1.

Les deux branches 10a et 10b du "U" formant le bras d'articulation 10 comportent respectivement un alésage latéral débouchant 22 dans lequel peut être logé un pivot 24 solidaire de la mâchoire fixe 2. Chaque pivot 24 est par exemple formé d'une vis engagée dans un filetage de la mâchoire fixe 2 et dont la tête 26, qui de préférence est rectifiée, est logée dans un des alésages 22, notamment par un ajustement glissant.

Par ailleurs, on remarquera que le "U" de la partie arrière de la mâchoire mobile 4 vient avantageusement coiffer la partie arrière correspondante de la mâchoire fixe 2.

Le corps de l'instrument, ainsi que la mâchoire fixe 2 qui vient de matière avec celui-ci, sont constitués de deux pièces P1 et P2 que l'on peut assembler selon leur longueur.

Ainsi, on comprendra que le montage du "U" sur la partie arrière de la mâchoire fixe 2 contribue au maintien en position fixement accolées des deux pièces P1 et P2.

Bien entendu, ces deux pièces peuvent comprendre sur leurs flancs latéraux respectifs des plots et des évidements correspondants, susceptibles de s'engager mutuellement pour procurer un effet de centrage.

Si l'instrument selon l'invention n'a pas pour vocation à être réutilisé, mais doit être jeté après l'intervention chirurgicale, une colle appropriée peut être disposée entre les deux flancs des pièces P1 et P2.

Toutefois, si cet instrument doit être nettoyé et stérilisé, et donc s'il doit être démonté, des vis noyées 28 qui sont engagées dans des filetages correspondants peuvent être prévues dans des régions appropriées du corps 1 de l'instrument et de la mâchoire fixe 2.

On précisera aussi que les deux vis 24 formant les pivots de la mâchoire mobile 4 peuvent être remplacées par un boulon qui traverse un alésage ménagé dans la partie arrière de la mâchoire fixe 2, et dont la tête et l'écrou sont convenablement rectifiés. La mise en place de ces éléments de construction est à la portée de l'homme de l'art, si bien que ces éléments n'ont pas été représentés ici de façon détaillée.

Le corps 1 de l'instrument et la mâchoire fixe 2 comportent intérieurement une première cavité longitudinale de guidage 30 servant à recevoir et à guider un outil 32 d'agrafage, cette cavité 30 qui comporte de préférence une section transversale rectangulaire, traversant de bout en bout le corps 1 et la mâchoire fixe 2.

Dès lors, la cavité 30 débouche, d'un côté, à l'extrémité arrière du corps 1 de l'instrument, et du côté opposé, dans un évidement 34 qui est ménagé dans la mâchoire fixe 2, jusqu'à son extrémité avant, et qui est de préférence plus haut que la cavité 30.

Ainsi, l'extrémité arrière 33 de l'outil 32 peut à son tour déboucher à l'extrémité arrière du corps 1 de l'instrument, tandis que l'extrémité avant de cet outil 32 porte une came 36 de déclenchement des agrafes, qui est logée dans l'évidement 34.

Cet évidement 34 débouche lui-même dans une fosse 38 ménagée dans la mâchoire fixe 2.

La fosse 38 est conformée pour recevoir un magasin d'agrafes qui peut être rechargé dans celle-ci.

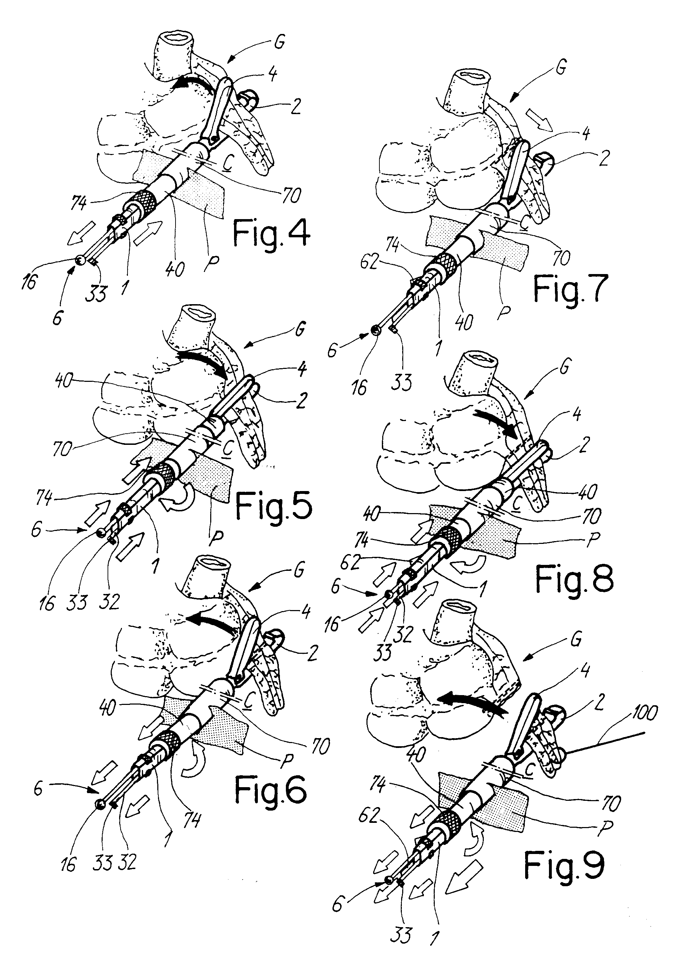

Ce magasin n'a pas été représenté. L'orientation des agrafes dans la fosse 38 est représentée schématiquement sur les figures 6 à 9, par rapport à une section d'un organe G à couper. Ces agrafes sont généralement prévues sur deux rangées parallèles décalées dans la longueur, et sont orientées parallèlement à l'instrument.

On remarquera que l'ensemble cavité 30 - évidement 34 - fosse 38 forme un orifice de section variable, apte à recevoir les moyens d'agrafage.

L'instrument de chirurgie selon l'invention comporte de plus des moyens de serrage des deux mâchoires 2 et 4 formant pince, notamment lorsqu'elles emprisonnent les tissus d'un organe.

Ces moyens de serrage sont constitués par un tube 40 monté coulissant autour du corps 1, coaxialement à celui-ci. Une extrémité avant d'appui 42 de ce tube 40 est apte à venir s'engager sur la partie arrière des mâchoires fixe 2 et mobile 4, lorsque ces mâchoires sont fermées, comme il est représenté sur la figure 1.

Cette extrémité avant 42 du tube 40 comporte intérieurement une région au moins partiellement troncônique 44 qui est conformée pour venir s'ajuster sur des régions partiellement troncôniques correspondantes 46 et 48, ménagées respectivement sur les parties arrières des mâchoires fixe 2 et mobile 4.

Ce tube de serrage 40 comporte de plus des filets intérieurs 50 prévus pour venir s'engager dans des secteurs extérieurement filetés 52 et 54 ménagés au moins partiellement sur le pourtour extérieur du corps 1.
La longueur du tube de serrage 40 est choisie de telle sorte que l'extrémité arrière de ce tube 40, qui comporte un renflement 74 venu de matière et formant bague de commande, puisse être accessible au chirurgien lorsque l'instrument est inséré dans un tube de trocart 70, l'instrument étant conformé pour être apte à coulisser dans ce tube 70.

On observera que le corps 1 et la pince 2, 4 lorsqu'elle est fermée, comportent en section transversale un pourtour extérieur partiellement circulaire, raccordé à des méplats latéraux 56, 58 pratiqués sur toute la longueur de l'instrument.

Ainsi, ces méplats 56, 58 forment chacun une face latérale dans laquelle peut être ménagée une rainure longitudinale 60 par exemple en forme de queue d'aronde.

Sur la figure 2, il n'a été représenté que deux rainures 60, étant bien entendu que plusieurs rainures peuvent être prévues sur une face 56, 58, voire sur les deux faces 56 et 58.

Cette rainure longitudinale 60 est destinée à recevoir un couteau 62 introduit depuis l'extrémité arrière du corps 1, et suffisamment long pour que sa lame 64 puisse, d'une part, atteindre la zone de pincement des tissus entre les deux mâchoires 2 et 4, tout en étant d'autre part commandée depuis l'extrémité arrière du corps 1 de l'instrument.

Les dimensions diamétrales du corps 1 de l'instrument, au niveau de ses régions cirulaires 57 et 59, sont inférieures aux dimensions diamétrales correspondantes des régions circulaires 61 et 63 de la pince formée par les mâchoires 2 et 4 réunies.

De ce fait, lorsque l'instrument est inséré dans le tube de guidage 70 qui traverse les tissus et qui est laissé en place lors de l'opération au niveau de la cavité abdominale, le tube de serrage 40 peut passer et coulisser librement entre le corps 1 et le tube 70, coaxialement à ceux-ci.

Qui plus est, pour faciliter la manipulation du corps 1 de l'instrument selon l'invention par un chirurgien, ce corps 1 comporte deux secteurs moletés démontables 72 et 73.

Ces secteurs 72 et 73 lorsqu'ils sont démontés permettent le coulissement vers l'arrière du tube de serrage 40, en vue du démontage complet de l'instrument.

En observant les mâchoires fixe 2 et mobile 4, on remarquera qu'elles comportent deux ergots saillants 76 et 78 entre lesquels peuvent venir se loger les tissus à agrafer.

Ces ergots 76 et 78 sont ménagés sur la mâchoire fixe 2 pour définir une zone d'agrafage et ils font saillie de celle-ci en direction de la mâchoire mobile 4. Ces ergots 76 et 78 s'étendent transversalement sur la mâchoire fixe 2, partiellement sur la largeur de cette dernière.

Ainsi, on remarquera qu'entre ces ergots 76, 78, et de part et d'autre de ceux-ci, s'étendent des plans de réception des tissus 80, 82.

La mâchoire mobile 4 comporte deux évidements 86 et 88 formés pour recevoir respectivement les ergots 76 et 78.

Entre ces évidements est prévue une platine 84 sur laquelle viendront se refermer, lors de la phase d'agrafage, les agrafes contenues dans la fosse 38.

La fosse 38 débouche entre les ergots 76, 78, en regard de la platine 84 de la mâchoire mobile 4, c'est-à-dire dans la zone d'agrafage des tissus.

Pour définir la distance de pincement des tissus entre les mâchoires fixe 2 et mobile 4, une butée 90 qui est fixe ou règlable est prévue à l'extrémité avant, de préférence, de la mâchoire mobile 4, en regard de l'extrémité correspondante de la mâchoire fixe 2. On remarquera par ailleurs qu'à l'extrémité avant de la mâchoire fixe est fixé (par collage, vissage ou analogue) un embout P3 d'une forme partiellement sphérique pour éviter de blesser des organes lors de l'indroduction de l'instrument dans la paroi abdominale, par l'intermédiaire du tube de trocart 70.

L'embout P3 ferme l'orifice de réception des moyens d'agrafage, et plus particulièrement il ferme l'évidement 34. Il sert ainsi d'élément de protection et de butée susceptible d'accueillir la pointe avant de la came 36 en fin de course, pour empêcher que celle-ci ne sorte de l'instrument et pour éviter toute blessure des tissus.

On a représenté sur les figures 4 à 9 l'instrument selon l'invention dans ses différentes phases de fonctionnement.

On remarquera que dans toutes ces phases opératoires, la partie arrière 74 du tube de serrage 40 (qui est extérieurement moletée), l'embout sphérique 16 (formant le moyen de commande 6 de la pince 2, 4), l'extrémité arrière 33 de l'outil 32, ainsi que le couteau 62 sont accessibles et manipulables par un chirurgien depuis l'extérieur de la cavité abdominale C qui est représentée partiellement sur les figures 4 à 9.

Le fonctionnement de l'instrument selon l'invention est le suivant :
Tout d'abord, un tube de trocart 70 est mis en place de façon classique pour pénétrer la cavité abdominale C, au travers de la paroi abdominale P représentée ici partiellement.

Ensuite, l'instrument selon l'invention, dont les mâchoires 2 et 4 sont fermées, est inséré à l'intérieur du tube 70. L'instrument dans son intégralité, à savoir les pinces 2, 4 puis le corps 1 entouré du tube de serrage 40, est inséré par coulissement à l'intérieur du tube de guidage 70 en direction de l'organe à opérer G.

Après que les mâchoires 2 et 4 aient passé l'extrémité avant du tube de guidage 70 (figure 4), le moyen de commande 6 de la mâchoire mobile 4 est tiré vers l'arrière afin d'ouvrir les mâchoires 2, 4 et d'amener entre elles les tissus de l'organe G.

Comme le représente la figuré 5, dès que les tissus sont logés dans la zone d'agrafage (ergots 76, 78 de la mâchoire 2), le moyen de commande 6 est poussé vers l'avant afin de fermer la mâchoire mobile 4 sur la mâchoire fixe 2, le tube de serrage 40 est poussé en direction des mâchoires qui sont dans leur position fermée, jusqu'à ce que les filets intérieurs 50 (figure 1) du tube de serrage 40 rencontrent les secteurs filetés 52, 54 du corps 1 de l'instrument.

A ce moment, on fait tourner le tube de serrage 40 pour engager les filets 50, 52 et 54, jusqu'à ce que les parties troncôniques 44 du tube de serrage 40 et 46, 48 des mâchoires 2, 4 viennent en contact et créent un couple resistant suffisant. Etant donné que l'angle de ces parties troncôniques est prévu pour être autoserrant, les mâchoires 2,4 sont immobilisées l'une contre l'autre et maintiennent fixement compressée entre elles une première section de l'organe G à agrafer.

Les tissus étant immobilisés, l'extrémité 33 de l'outil 32 est poussée vers l'avant afin que la came 36 vienne déclencher les agrafes contenues dans la mâchoire fixe 2.

Ensuite, comme représenté sur la figure 6, l'outil 32 est tiré vers l'arrière, le tube de serrage 40 est dévissé, puis il est convenablement tiré jusqu'à libérer les mâchoires 2 et 4. Le moyen de commande 6 est alors tiré pour ouvrir la mâchoire mobile 4.

L'instrument peut alors être retiré de la cavité abdominale pour être rechargé (ou remplacé par un deuxième instrument identique, chargé).

Une première rangée d'agrafes étant posée, on déplace l'instrument vers le côté, généralement en l'obliquant sensiblement, jusqu'à disposer une nouvelle partie des tissus encre les mâchoires 2 et 4 (figure 7).

La pince 2, 4 étant convenablement positionnée (une partie des tissus étant de nouveau dans la zone d'agrafage), on la referme en actionnant de nouveau les moyens de commande 6 vers l'avant (figure 8), puis on la bloque en avançant et en vissant le tube de serrage 40 pour venir faire coopérer les parties troncôniques autoserrantes. Une nouvelle partie de l'organe G étant fortement immobilisée, on actionne de nouveau les moyens d'agrafage en poussant l'outil 32.

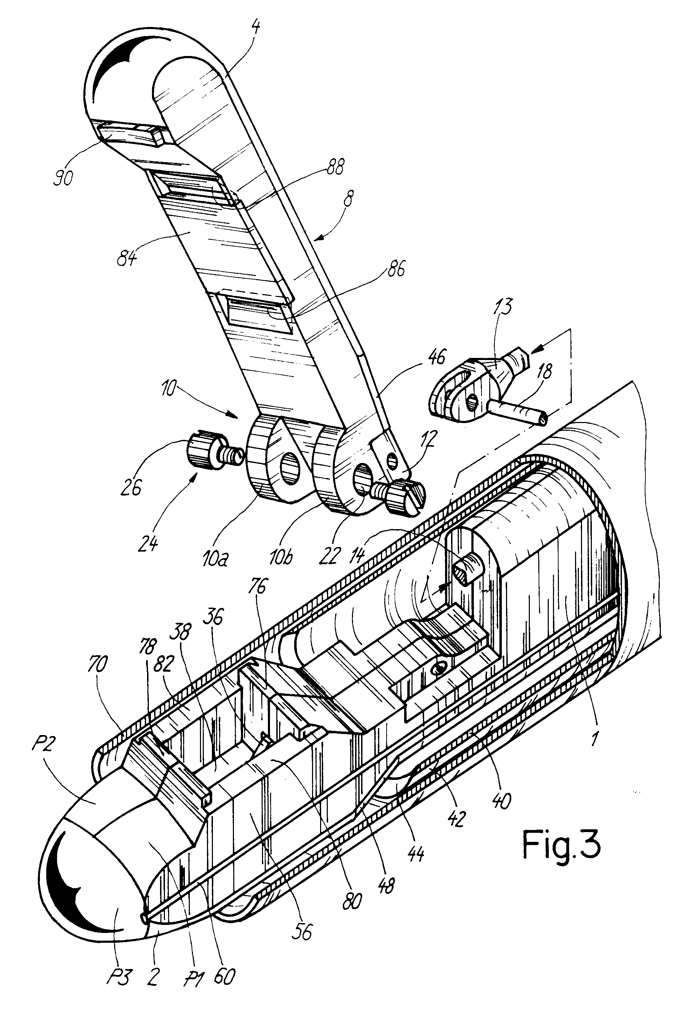

Une deuxième rangée d'agrafes étant posée, on actionne le couteau 62 qui est de préférence prépositionné sur le côté du corps 1. Le couteau 62 peut être amené en cours d'opération sur l'instrument. Une partie de guidage du couteau 62 étant engagée dans la rainure latérale 60 (figures 1 et 3) on fait coulisser ce couteau latéralement sur le corps 1, à l'intérieur du tube de serrage 40 pour amener la partie tranchante 64 de ce couteau entre les deux rangées d'agrafes préalablement posées.

Comme représenté sur la figure 9, le couteau 62 est ensuite tiré vers l'arrière. Le tube de serrage 40 est dévissé par action sur la bague 74, puis il est tiré, et enfin les moyens de commande 6 de la mâchoire mobile 4 ainsi que l'outil 32 sont tirés vers l'extérieur. L'ablation étant réalisée, la partie coupée de l'organe G est saisie par une pince 100 qui a été de même introduite par un tube de trocart, au travers de la paroi abdominale. Les deux mâchoires 2, 4 sont ensuite ramenées l'une vers l'autre par actionnement des moyens de commande 6 et l'instrument de chirurgie selon l'invention est sorti de la cavité abdominale C au travers du tube de guidage 70. Le tube de guidage 70 est ensuite enlevé de la paroi abdominale P. On remarquera donc que dans ce procédé on a tout d'abord posé une première rangée d'agrafes, puis on a déplacé l'instrument pour poser une deuxième rangée à côté de la première, ce qui a permis au chirurgien de s'assurer, à chaque stade de l'opération, de la qualité de l'agrafage. Ensuite, on a coupé entre les deux rangées d'agrafes pour détacher la partie défaillante de l'organe G. Ainsi, on a évité tout risque d'épanchement à l'intérieur de la cavité abdominale C puisqu'il a été possible de vérifier la qualité de l'agrafage.

Par ailleurs, étant donné la construction particulièrement compacte du corps 1 de l'instrument et des mâchoires 2 et 4, l'instrument peut être manipulé avec précision par le chirurgien. De plus, les moyens de serrage tels qu'ils sont conçus, offrent un serrage suffisant des deux mâchoires l'une sur l'autre, ce qui contribue bien entendu à accroître l'efficacité de l'agrafage.

## Revendications

1. Un instrument de chirurgie pour chasser des éléments de fixation dans le tissu d'un corps, comprenant:
(a) un corps allongé (1) à une extrémité distale, qui définit une partie de corps endoscopique qui comprend une première mâchoire allongée fixe (2) et comporte au moins deux rangées des agrafes chirurgicales le long de la longueur du corps, et à une extrémité proximale, qui définit une partie de corps de commande à être actionnée par un chirurgien;
(b) une deuxième mâchoire allongée (4) montée à un mouvement pivotant sur ledit corps autour d'un pivot à l'extrémité proximale de la mâchoire, les longueurs desdites première et deuxième mâchoires étant alignées l'une sur l'autre et sur l'orientation de la longueur du corps, entre une position d'ouverture, à laquelle l'extrémité distale de ladite deuxième mâchoire est écartée de l'extrémité distale de la première mâchoire, et une position de fermeture, à laquelle ladite deuxième mâchoire est fermée contre ladite mâchoire fixe, en alignement sur la dernière; et
(c) des moyens de commande qui s'étendent au travers de la longueur dudit corps, de l'extrémité proximale à l'extrémité distale du corps, pour la manipulation desdites mâchoires et desdites agrafes à son extrémité distale, et caractérisé en ce que lesdits moyens de commande comprennent:
(i) des premiers moyens de commande à un premier élément de commande allongé (6), qui s'étend de la partie de corps de manipulation et qui est mobile entre une première position et une deuxième position afin de commander la position pivotée de ladite deuxième mâchoire, relative à ladite première mâchoire fixe;
(ii) et en ce que lesdits premiers moyens de commande comprennent de plus un moyen d'arrêt tubulaire (40) mobile du côté distal, d'une position de relâchement, qui permet le mouvement pivotant de ladite deuxième mâchoire dans sa position d'ouverture, à une position de blocage, à laquelle il porte contre ladite deuxième mâchoire (4) en empêchant un mouvement pivotant de ladite deuxième mâchoire en départ de sa position de fermeture vers sa position d'ouverture;
(iii) et qu'il comprend des deuxièmes moyens de commande à un deuxième élément de commande allongé (32) qui s'étend au travers desdits moyens d'arrêt (40) et qui est mobile, en sens longitudinal, après le blocage de la deuxième mâchoire, de façon à appliquer lesdites deux rangées des agrafes chirurgicales au tissu du corps, qui est pincé entre lesdites mâchoires.

2. Instrument selon la revendication 1, caractérisé en ce que la mâchoire mobile (4) a la forme d'un levier coudé dont un bras (8) forme la mâchoire proprement dite, tandis que l'autre bras (10) est articulé de préférence à la jonction entre la mâchoire fixe (2) et l'extrémité avant du corps (1) de l'instrument.

3. Instrument selon la revendication 2, caractérisé en ce que le moyen de commande (6) de la mâchoire mobile (4) est constitué par une tige rigide attelée à cette mâchoire (4), à proximité de la jonction entre les deux bras précités (8, 10), cette tige (14) étant logée à l'intérieur du corps (1) de l'instrument dans lequel elle peut coulisser.

4. Instrument selon l'une des revendications 1 à 3, caractérisé en ce que la partie arrière de la mâchoire mobile (4) présente, au moins en partie, la forme d'un "U" renversé qui coiffe la partie arrière de la mâchoire fixe (2), et dont chaque branche (10a, 10b) porte respectivement un orifice latéral débouchant (22) dans lequel est logé un pivot (24) solidaire de la mâchoire fixe (2).

5. Instrument selon la revendication 4, caractérisé en ce que les deux pivots (24) sont formés par des vis dont la tête (26) est logée dans l'un des orifices correspondants (22) des branches du "U", avec un ajustement glissant.

6. Instrument selon l'une quelconque des revendications précédentes, caractérisé en ce que les moyens de serrage sont constitués par un tube (40) monté coulissant autour du corps (1) de l'instrument et dont une extrémité d'appui avant (42) présente intérieurement une région au moins partiellement troncônique (44) qui est susceptible de venir s'ajuster sur des régions troncôniques correspondantes (46, 48) ménagées sur la partie arrière des mâchoires respectivement fixe (2) et mobile (4).

7. Instrument selon la revendication 6, caractérisé en ce que le tube de serrage (40) comporte des filets intérieurs (50) aptes à venir s'engager dans des secteurs extérieurement filetés (52, 54), ménagés au moins partiellement sur le pourtour extérieur du corps (1) de l'instrument.

8. Instrument selon l'une quelconque des revendications précédentes, caractérisé en ce que le corps (1) de l'instrument et les mâchoires fixe (2) et mobile (4) comportent un pourtour au moins partiellement circulaire, associé à deux méplats longitudinaux (56, 58).

9. Instrument selon la revendication 8, caractérisé en ce que le corps (1) comporte au niveau de ses régions circulaires (57, 59) des dimensions diamétrales extérieures inférieures à celles des régions circulaires (61, 63) de la pince formée par les mâchoires (2, 4) réunies, pour permettre le passage du tube de serrage (40) entre le corps (1) de l'instrument et un tube de guidage ou trocart (70).

10. Instrument selon l'une quelconque des revendications précédentes, caractérisé en ce que la mâchoire fixe (2) vient de matière avec le corps (1) de l'instrument.

11. Instrument selon l'une quelconque des revendications précédentes, caractérisé en ce que le corps (1) de l'instrument et la mâchoire fixe (2) sont formés de deux pièces (P1, P2) assemblées selon leur longueur.

12. Instrument selon la revendication 11, caractérisé en ce que les deux pièces (P1, P2) formant le corps de l'instrument et la mâchoire fixe (2) sont maintenues fixement accolées au moins par leur engagement dans le "U" de montage de la partie arrière de la mâchoire mobile (4).

13. Instrument selon l'une quelconque des revendications précédentes, caractérisé en ce le corps (1) de l'instrument et la mâchoire fixe (2) comportent un premier orifice longitudinal (30, 34), de préférence rectangulaire, dans laquelle peut coulisser un outil (32) dont une extrémité (33) débouche à l'extrémité arrière dudit corps (1), tandis que l'autre extrémité porte une came (36) de déclenchement des agrafes.

14. Instrument selon la revendication 13, caractérisé en ce que l'orifice précité (30, 34) est de plus formé par une fosse (38) ménagée dans la mâchoire fixe (2) et apte à recevoir un magasin d'agrafes rechargeable.

15. Instrument selon la revendication 14, caractérisé en ce que la fosse (38) débouche dans une zone d'agrafage, en regard d'une platine (84) de la mâchoire mobile (4), destinée à refermer les agrafes.

16. Instrument selon l'une des revendications précédentes, caractérisé en ce que le corps (1) de l'instrument et la mâchoire fixe (2) comportent au moins une rainure de guidage longitudinale (60) servant à maintenir un couteau (62), cette rainure (60) étant ménagée sur au moins une face latérale (56, 58) de l'ensemble corps (1) - mâchoire fixe (2).

17. Instrument selon l'une quelconque des revendications précédentes, caractérisé en ce que l'une des mâchoires, de préférence la mâchoire fixe (2), comporte au moins deux ergots (76, 78) entre lesquels viennent se loger les tissus de l'organe (G) à couper.

18. Instrument selon l'une quelconque des revendications précédentes, caractérisé en ce que la mâchoire mobile (4), de préférence, comporte une butée (90) permettant de déterminer la distance de pincement des tissus, entre les deux mâchoires.

19. Instrument selon l'une quelconque des revendications précédentes, caractérisé en ce que l'extrémité arrière du corps de l'instrument comporte deux secteurs moletés démontables (73, 74) permettant le maintien de l'instrument ainsi que le démontage par coulissement arrière du tube de serrage (40), sur ledit corps (1).

## Claims

1. A surgical instrument for securing fixing members in body tissue, comprising:
a) an elongated body (1) which at one distal extremity defines a part of an endoscope body comprising a first fixed elongated jaw (2) and comprising at least two rows of surgical staples along the length of the body, and which at a proximal extremity defines a part of a control body operated by a surgeon,
b) a second elongated jaw (4) mounted so as to pivot on the said body about a pivot at the proximal extremity of the jaw, the lengths of the said first and second jaws being aligned with each other and along the orientation of the length of the body between an open position in which the distal extremity of the said second jaw is separated from the distal extremity of the first jaw, and a closed position in which the said second jaw is closed against the said fixed jaw and is aligned therewith, and
c) control means which extend throughout the length of the said body, from the proximal extremity of the body to the distal extremity, for manipulation of the said jaws and the said staples at its distal extremity, and characterised in that the said control means comprise:
(i) first control means for a first elongated control member (6) which extends from the control body part and which moves between a first position and a second position so as to control the pivoted position of the said second jaw relative to the said first fixed jaw,
(ii) and in that the said first control means also comprise tubular stop means (40) which can be moved from the distal side from a released position which allows pivoting movement of the said second jaw into its open position to a locked position in which it bears against the said second jaw (4) preventing pivoting movement of the said second jaw from its closed position to its open position, (iii) and which comprises second control means for a second elongated control member (32) which extends across the said stopping means (40) and which can move in a longitudinal direction after the second jaw has been locked in such a way as to apply the said two rows of surgical staples to the body tissue which is pinched between the said jaws.

2. An instrument according to claim 1, characterised in that the moving jaw (4) has the form of an angled lever of which one arm (8) forms the jaw proper, while the other arm (10) is hinged preferably at the junction between the fixed jaw (2) and the forward end of the body (1) of the instrument.

3. An instrument according to claim 2, characterised in that the control means (6) for the moving jaw (4) comprises a rigid rod linked to that jaw (4) in the vicinity of the junction between the two aforesaid arms (8, 10), this rod (14) being housed within the body (1) of the instrument, in which it may slide.

4. An instrument according to any of claims 1 to 3, characterised in that the rear part of the moving jaw (4) has at least in part an upside-down "U" shape covering the rear part of the fixed jaw (2), each limb (10a, 10b) of which respectively bears a lateral opening (22) in which is housed a pivot (24) which is integral with the fixed jaw (2).

5. An instrument according to claim 4, characterised in that the two pivots (24) are formed by screws whose heads (26) are housed in one of the corresponding openings (22) in the limbs of the "U" with a sliding fit.

6. An instrument according to any one of the foregoing claims, characterised in that the locking means comprise a tube (40) which is slidably mounted around the body (1) of the instrument and of which one forward bearing extremity (42) has internally an at least partly tapering region (44) which can be made to fit against corresponding tapering regions (46, 48) provided in the rear parts of the fixed (2) and moving (4) jaws respectively.

7. An instrument according to claim 6, characterised in that the locking tube (40) has internal threads (50) which can engage with externally threaded sections (52, 54) provided over at least part of the external perimeter of the body (1) of the instrument.

8. An instrument according to any one of the foregoing claims, characterised in that the body (1) of the instrument and the fixed (2) and moving (4) jaws have an at least partly circular perimeter associated with two longitudinal flats (56, 58).

9. An instrument according to claim 8, characterised in that the body (1) has external diametral dimensions in the vicinity of its circular regions (57, 59) which are smaller than those of the circular regions (61, 63) of the pincer formed by the closed jaws (2, 4) to permit the passage of a locking tube (40) between the body (1) of the instrument and a guide tube or trocar (70).

10. An instrument according to any one of the foregoing claims, characterised in that the fixed jaw (2) is of one piece with the body (1) of the instrument.

11. An instrument according to any one of the foregoing claims, characterised in that the body (1) of the instrument and the fixed jaw (2) are formed of two parts (P1, P2) assembled in line with each other.

12. An instrument according to claim 11, characterised in that the two pieces (P1, P2) forming the body of the instrument and the fixed jaw (2) are firmly secured alongside each other at least as a result of their engaging with the "U" mounting the rear part of the moving jaw (4).

13. An instrument according to any one of the foregoing claims, characterised in that the body (1) of the instrument and the fixed jaw (2) include a first longitudinal opening (30, 34), which is preferably rectangular, in which a tool (32), of which one end (33) projects from the rear extremity of the said body (1), can slide, while the other extremity bears a cam (36) for releasing staples.

14. An instrument according to claim 13, characterised in that the aforesaid opening (30, 34) is also formed by a channel (38) provided in the fixed jaw (2) and is capable of receiving a rechargeable magazine of staples.

15. An instrument according to claim 14, characterised in that the channel (38) opens into a stapling zone opposite a plate (84) on the moving jaw (4) which is designed to close the staples.

16. An instrument according to one of the foregoing claims, characterised in that the body (1) of the instrument and the fixed jaw (2) incorporate at least one longitudinal guide groove (60) which is used to hold a knife (62), this groove (60) being located on at least one lateral side (56, 58) of the body (1) - fixed jaw (2) assembly.

17. An instrument according to any one of the foregoing claims, characterised in that one of the jaws, preferably the fixed jaw (2), incorporates at least two spurs (76, 78) between which the tissues of the organ (G) which is to be cut are held.

18. An instrument according to any one of the foregoing claims, characterised in that the moving jaw (4) preferably incorporates a stop (90) which can be used to determine the distance over which tissues are pinched between the two jaws.

19. An instrument according to any one of the foregoing claims, characterised in that the rear extremity of the body of the instrument incorporates two removable knurled sections (73, 74) by which the instrument may be held together and by which it may be dismantled by sliding backwards the locking tube (10) over the said body (1).

## Patentansprüche

1. Chirurgisches Instrument zum Eintreiben von Befestigungselementen in das Gewebe eines Körpers, umfassend:
(a) einen langgestreckten Körper (1) mit einem distalen Ende, das einen Teil eines Endoskopkörpers begrenzt, der eine erste, langgestreckte, feste Backe (2) umfaßt und mindestens zwei Reihen chirurgischer Klammern in Längsrichtung am Körper entlang aufweist, und mit einem proximalen Ende, das einen Teil eines vom Chirurgen zu bedienenden Betätigungskörpers begrenzt;
(b) eine zweite langgestreckte Backe (4), die für eine Kippbewegung um einen Drehzapfen am proximalen Ende der Backe am besagten Körper angebracht ist, wobei die Längen der besagten Backen, der ersten und der zweiten, übereinander und mit der Längsrichtung des Körpers ausgerichtet sind und die Kippbewegung zwischen einer Offenstellung, in der das distale Ende der besagten zweiten Backe vom distalen Ende der ersten Backe abgespreizt ist, und einer Schließstellung, in der die besagte zweite Backe gegen die besagte feste Backe geschlossen und dabei mit der letzteren ausgerichtet ist; und
(c) Betätigungsmittel für die Betätigung der besagten Backen und der besagten Klammern am distalen Ende des Körpers, die sich durch die Länge des besagten Körpers vom proximalen Ende bis zum distalen Ende des Körpers erstrecken, dadurch gekennzeichnet, daß die besagten Betätigungsmittel umfassen:
(i) erste Betätigungsmittel mit einem ersten langgestreckten Betätigungselement (6), das sich vom Bedienungsteil des Körpers aus erstreckt und zwischen einer ersten Stellung und einer zweiten Stellung bewegbar ist, um die gekippte Stellung der besagten zweiten Backe relativ zu der besagten ersten, festen Backe zu steuern;
(ii) und dadurch, daß die besagten ersten Betätigungsmittel außerdem ein röhrenförmiges Sperrorgan (40) umfassen, das von der distalen Seite aus einer Lösestellung, welche die Kippbewegung der besagten zweiten Backe in deren Offenstellung gestattet, in eine Sperrstellung bewegbar ist, in der es gegen die besagte zweite Backe (4) drückt, wobei es eine Kippbewegung der besagten zweiten Backe aus ihrer Schließstellung in ihre offenstellung verhindert;
(iii) und daß es zweite Betätigungsmittel mit einem zweiten langgestreckten Betätigungselement (32) umfaßt, das sich durch die besagten Sperrmittel (40) erstreckt und nach Blockierung der zweiten Backe derart in Längsrichtung bewegbar ist, daß es die beiden besagten Reihen chirurgischer Klammern am Gewebe des Körpers anbringt, das zwischen den besagten Backen festgehalten wird.

2. Instrument nach Anspruch 1, dadurch gekennzeichnet, daß die bewegliche Backe (4) die Form eines gekrümmten Hebels hat, dessen einer Arm (8) die eigentliche Backe bildet, während der andere Arm (10) vorzugsweise an der Verbindungsstelle zwischen der festen Backe (2) und dem Vorderende des Körpers (1) des Instruments angelenkt ist.

3. Instrument nach Anspruch 2, dadurch gekennzeichnet, daß das Betätigungsmittel (6) der beweglichen Backe (4) aus einer starren Stange besteht, die an dieser Backe (4) nahe der Verbindungsstelle zwischen den beiden erwähnten Armen (8, 10) angekuppelt ist, wobei sich diese Stange (14) im Inneren des Körpers (1) des Instruments befindet, in dem sie sich verschieben kann.

4. Instrument nach einem der Ansprüche 1 bis 3, dadurch gekennzeichnet, daß der hintere Teil der beweglichen Backe (4) mindestens teilweise die Form eines umgekehrten "U" hat, das auf dem hinteren Teil der festen Backe (2) sitzt und dessen beide Arme (10a, 10b) je eine seitliche Öffnung (22) aufweisen, die einen mit der festen Backe (2) fest verbundenen Drehzapfen (24) aufnimmt.

5. Instrument nach Anspruch 4, dadurch gekennzeichnet, daß die beiden Drehzapfen (24) aus Schrauben bestehen, deren Kopf (26) in einer der entsprechenden Öffnungen (22) der Arme des "U" mit Gleitsitz ruht.

6. Instrument nach einem beliebigen der vorangehenden Ansprüche, dadurch gekennzeichnet, daß die Verriegelungsmittel aus einem Rohr (40) bestehen, das um den Körper (1) des Instruments verschiebbar angebracht ist und dessen vorderes Druckende (42) innen einen mindestens teilweise kegelstumpfförmigen Bereich (44) aufweist, der in der Lage ist, sich an entsprechenden kegelstumpfförmigen Bereichen (46, 48) auszurichten, die am hinteren Teil der festen (2) bzw. beweglichen Backe (4) ausgebildet sind.

7. Instrument nach Anspruch 6, dadurch gekennzeichnet, daß das Verriegelungsrohr (40) Innengewinde (50) aufweist, die in der Lage sind, in Abschnitte mit Außengewinde (52, 54) einzugreifen, die mindestens teilweise auf dem äußeren Umfang des Körpers (1) des Instruments ausgebildet sind.

8. Instrument nach einem beliebigen der vorangehenden Ansprüche, dadurch gekennzeichnet, daß der Körper (1) des Instruments und die feste (2) und die bewegliche Backe (4) eine teilweise kreisförmige Umfangslinie aufweisen, die mit zwei Abflachungen (56, 58) in Längsrichtung verbunden ist.

9. Instrument nach Anspruch 8, dadurch gekennzeichnet, daß der Körper (1) des Instruments in Höhe dieser kreisförmigen Bereiche (57, 59) äußere Durchmesser aufweist, die kleiner sind, als diejenigen der kreisförmigen Bereiche (61, 63) der Zange, die durch die Backen (2, 4) gemeinsam gebildet wird, um den Durchtritt des Verriegelungsrohrs (40) zwischen dem Körper (1) des Instruments und einem Führungs- oder Trokarrohr (70) zu ermöglichen.

10. Instrument nach einem beliebigen der vorangehenden Ansprüche, dadurch gekennzeichnet, daß die feste Backe (2) an den Körper (1) des Instruments angeformt ist.

11. Instrument nach einem beliebigen der vorangehenden Ansprüche, dadurch gekennzeichnet, daß der Körper (1) des Instruments und die feste Backe (2) aus zwei in ihrer Längsrichtung zusammengesetzten Teilen (P1, P2) bestehen.

12. Instrument nach Anspruch 11, dadurch gekennzeichnet, daß die beiden den Körper des Instruments und die feste Backe (2) bildenden Teile (P1, P2) mindestens dadurch fest aneinandergefügt gehalten werden, daß sie in das "U" zur Montage des hinteren Teils der beweglichen Backe (4) eingefügt sind.

13. Instrument nach einem beliebigen der vorangehenden Ansprüche, dadurch gekennzeichnet, daß der Körper (1) des Instruments und die feste Backe (2) eine erste, vorzugsweise rechteckige Öffnung in Längsrichtung (30, 34) aufweisen, in der sich ein Werkzeug (32) verschieben kann, dessen eines Ende (33) am hinteren Ende des besagten Körpers (1) austritt, während das andere Ende einen Nocken (36) zum Auslösen der Klammern trägt.

14. Instrument nach Anspruch 13, dadurch gekennzeichnet, daß die vorgenannte Öffnung (30, 34) außerdem eine Rinne (38) umfaßt, die in der festen Backe (2) ausgebildet und in der Lage ist, ein bestückbares Klammermagazin aufzunehmen.

15. Instrument nach Anspruch 14, dadurch gekennzeichnet, daß die Rinne (38) in einen Klammerbereich mündet, der einer Platte (84) der beweglichen Backe (4) gegenüberliegt, die zum Schließen der Klammern bestimmt ist.

16. Instrument nach einem der vorangehenden Ansprüche, dadurch gekennzeichnet, daß der Körper (1) des Instruments und die feste Backe (2) mindestens eine Führungsnut in Längsrichtung (60) aufweisen, die dazu dient, ein Messer (62) zu halten, wobei diese Nut (60) in mindestens einer Seitenfläche (56, 58) der Einheit Körper (1) - feste Backe (2) ausgebildet ist.

17. Instrument nach einem beliebigen der vorangehenden Ansprüche, dadurch gekennzeichnet, daß eine der Backen, vorzugsweise die feste Backe (2), mindestens zwei Vorsprünge (76, 78) aufweist, zwischen denen das zu schneidende Gewebe des Organs (G) aufgenommen wird.

18. Instrument nach einem beliebigen der vorangehenden Ansprüche, dadurch gekennzeichnet, daß die bewegliche Backe (4) vorzugsweise einen Anschlag (90) aufweist, der es gestattet, den Abstand festzulegen, in dem Gewebe zwischen den beiden Backen gefaßt wird.

19. Instrument nach einem beleibigen der vorangehenden Ansprüche, dadurch gekennzeichnet, daß das hintere Ende des Körpers des Instruments zwei demontierbare, gerändelte Abschnitte (73, 74) aufweist, die das Halten des Instruments, sowie die Demontage durch Verschieben des Verriegelungsrohrs (40) auf dem besagten Körper (1) nach hinten ermöglichen.
